# EUROPEAN PATENT APPLICATION

(11) **EP 3 308 770 A1**
(43) Date of publication of application: **18.04.2018**
(21) Application number: 17205196.3
(22) Date of filing: 14.04.2014
(51) Int. Cl.: A61K 9/08, A61K 9/19, A61K 9/14, A61K 47/12, A61K 47/18, A61K 47/20, A61K 31/519

(54) **PEMETREXED FORMULATION**

(30) Priority: 12.04.2013 IS 50051; 14.11.2013 IS 50068
(62) Divisional of application: 14723490.0
(71) Applicant: Actavis Group PTC EHF, 220 Hafnarfjordur (IS)
(72) Inventor: ROTARU, Maria, 021213 Bucharest 2 (RO)
(74) Representative: Arnason Faktor

(57) **Abstract**

The invention provides stable formulations of pemetrexed for infusion. The formulations are based on using pemetrexed diacid and certain selected suitable stabilising basic amine compounds that provide counter ions to the pemetrexed diacid, forming base addition salts. The formulations can be dried powder formulations to be reconstituted as liquid concentrate formulations or directly in ready-to-use infusion solutions, or they can be liquid formulations, most suitably concentrates to be diluted in infusion solution prior to use. The suitable basic amine addition compounds according to the invention are one or more of diethanolamine, tris-(hydroxymethyl)aminomethane and meglumine.

## Description

### FIELD OF INVENTION

The invention is within the field of pharmaceutical formulations and specifically concerns new solid and liquid stable formulations of pemetrexed.

### TECHNICAL BACKGROUND AND PRIOR ART

Pemetrexed is a chemotherapy drug and is used in the treatment of malignant pleural mesothelioma and non-small cell lung cancer. Pemetrexed has the chemical name (2*S*)-2-{[4-[2-(2-amino-4-oxo-1,7-dihydro pyrrolo[2,3-*d*]pyrimidin-5-yl)ethyl]benzoyl]amino}-pentanedioic acid. The structural formula of the diacid is shown in Formula (I).

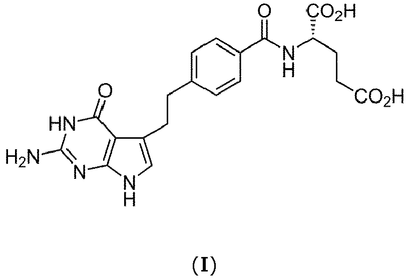

Pemetrexed is in the class of chemotherapy drugs called folate antimetabolites. By inhibiting thymidylate synthase (TS), dihydrofolate reductase (DHFR), and glycinamide ribonucleotide formyl transferase (GARFT), and hence the formation of precursor purine and pyrimidine nucleotides, Pemetrexed prevents the formation of DNA and RNA, which are required for the growth and survival of both normal cells and cancer cells.

The commercially-available product comprises the pemetrexed disodium salt and is sold under the trade name ALIMTA (Eli Lilly), is supplied as a sterile lyophilised powder in single-dose vials to be dissolved for intravenous infusion. The product formulation contains in addition to the active ingredient mannitol, and may contain hydrochloric acid and/ sodium hydroxide to adjust pH.

Pemetrexed is disclosed in EP0432677. Different crystalline forms and/or amorphous form are described in EP1259513, EP2072518 and WO2008124485. Pemetrexed diacid and its preparation is disclosed in US 5,416,211, US 6,262,262, and WO 2008021410. Certain formulations of pemetrexed are disclosed in WO 2012015810.

The relatively rapid formation of degradants is one of the factors which has prevented aqueous pemetrexed formulations having long-term stability from being commercially available. It has shown to be a challenge to formulate pemetrexed due to stability issues. New alternative pemetrexed formulations with improved stability would be appreciated.

### SUMMARY OF INVENTION

The present invention provides stable formulations of pemetrexed for infusion. The formulations are based on using pemetrexed diacid and certain selected suitable stabilising base compounds. The formulations can be dried powder formulations to be reconstituted as liquid concentrate formulations or directly in ready-to-use infusion solutions, or they can be liquid formulations, most suitably concentrates to be diluted in infusion solution prior to use. The suitable base addition compounds according to the invention is preferably a basic organic amine and most preferably one or more of diethanolamine, tris(hydroxymethyl)-aminomethane and meglumine.

### DETAILED DESCRIPTION

Investigations by the inventors showed that several tested counter ions failed to provide sufficient stable and/or soluble salts of pemetrexed. As the compound is administered by infusion, it must be in a form ready to be dissolved (if solid) or diluted (if liquid) in an infusion liquid, or be provided as ready for infusion. Solid formulations must be such that the entire contents are readily soluble leaving essentially no solid particles and providing all the active ingredient as homogeneously dissolved compound.

Liquid formulations do not require reconstitution in liquid form by medical personel but introduces stability challenges, as the active compound is more sensitive to degradation in aqueous environment.

The inventors surprisingly found that certain specific organic bases proved suitable and have provided highly stable formulations. Accordingly, the formulations use the diacid form of pemetrexed in combination with an organic base selected from diethanolamine, tris(hydroxymethyl)aminomethane and meglumine, or a combination of two or three of the base compounds. Addition salts of the acid and base are generally formed in solution and then preferably subjected to lyophilisation, and either provided as lyophilised powder or dissolved as liquid concentrate.

Pharmaceutical formulations according to the invention, and hereby in particular the liquid concentrate formulations, are generally preferred because they are highly stable and show only minimal decomposition of the active ingredient during storage. Different forms of the active compound are not readily soluble and the inventors tested several salt forms which failed to result in sufficiently and readily soluble form of the active compound. The present formulations have good solubility and prove easy to mix, lyophilise and reconstitute, and accordingly can be provided either as lyophilised powder forms to be reconstituted, or dissolved in aqueous solutions, that can be readily diluted prior to use.

However, liquid concentrate formulations do not need to be dissolved prior to use and are therefore preferred because they provide enhanced convenience in handling and administration.

The addition salts formed in the formulations of the invention are pharmaceutically acceptable and suitable for the relatively high doses administered of the active compound (typically 500 mg/m2, which for an average adult corresponds to 1100 mg of the active compound, typically administered with IV infusion in 10 minutes on day 1 of a 21-day cycle). These high doses exclude from use counter ions such as potassium, which would be toxic in such high amount. The organic base compounds however are quite suitable for such high dosing of addition salts of the active compounds and thereby provide and added benefit over other potential salts and prior art suggestions.

As mentioned above, liquid formulations have so far not been commercially available, but with the present invention it is possible to manufacture and sell the drug in easy-to-use liquid concentrate formulations. The formulations only need to be diluted into an IV infusion solution and the caregiver need not be concerned about whether all particles have been dissolved, clog tubes etc. This provides a great advantage over prior art solid formulations that must be dissolved prior to use.

Tris(hydroxymethyl)aminomethane shown in formula (II) is also referred to as TRIS, tromethamine and trisamine. It is widely used as buffer component. It is a primary amine and a weak base with a pKₐ of 8,07 at 25°C.

Meglumine is shown if formula (III), its chemical name is (2R,3R,4R,5S)-6-(Methylamino)-hexane-1,2,3,4,5-pentol, also referred to as emthylglucamin, 1-Deoxy-1-(methylamino)-D-glucitol or *N*-Methylsorbitylamine; it is an amino sugar derived from sorbitol.

Diethanolamine shown in formula (IV) is also referred to as DEA or DEOA, iminodiethanol, N-ethylethanamine, 2,2'-dihydroxydiethylamine. It is a polyfunctional compound miscible in water, being a secondary amine and a diol, and acts as a weak base.

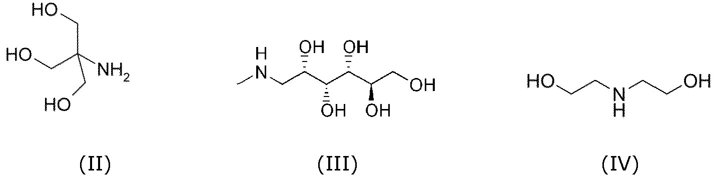

The pharmaceutical formulation may in useful embodiments be provided as liquid concentrate formulations, to be diluted just prior to use in an infusion solution. The liquid concentrate may in some embodiment comprise the active diacid compound in a concentration such as from about 5 mmolar to about 200 mmolar, which corresponds to about 2 to about 80 mg/mL. More preferably the concentration is in the range from about 5 to about 50 mg/mL, such as from about 10 to about 40 mg/mL, such as in the range from about 20-30 mg/mL, such as about 20 mg/mL, about 30 mg/mL or about 25 mg/mL (about 58 mmolar). The liquid concentrate is preferably provided in one-dose vials, with sufficient total amount of the active ingredient for a single patient dose, this means that each vial is for single use, opened and mixed into infusion solution.

In other embodiments the pharmaceutical formulations are provided as solid formulations, ready to be solubilised and reconstituted. The solid dry formulations of the invention are conveniently prepared by drying a concentrate solution such as typically by freeze-drying. The term freeze-drying as used herein is synonymous with the terms lyophilisation and cryodesiccation, and generally refers to a process of freezing material and then reducing surrounding pressure to allow frozen water in the material to sublimate directly from the solid phase to the gas phase.

The formulation may comprise one of the mentioned organic basic amine compounds or a combination of two or three of said compounds. In preferred embodiments, the total amount of the one or more basic organic amine is in the range corresponding to 0.3 to 2.0 mg per mg of pemetrexed diacid, and more preferably in the range in the range 0.5 to 1.5 mg per mg of pemetrexed diacid, such as about 0.5 mg, 0.6 mg, 0.7 mg, 0.8 mg 0.9 or 1.0 mg per mg of pemetrexed diacid. In other embodiments the amount of the organic amine is more than the amount of pemetrexed, such as in the range 1.0 to 2.0 mg per mg of pemetrexed diacid, or in the range 1.0 to 1.5 mg per mg of pemetrexed diacid, such as about 1.10 or 1.20 or 1.22 mg per mg of pemetrexed diacid.

In certain embodiments, the amount of the base is based on molar ratio, preferably the amount of base is in the range of about 1.0 to about 8.0 mol per mol of pemetrexed, such as in the range about 1.7 to 5.3, such as in the range 3.5 to about 5.3, such as in the range 4.9 to 5.0. Preferably the base present in these amounts is trisamine.

In certain preferred embodiments the organic base is tris(hydroxymethyl)aminomethane by itself, suitably in an amount as mentioned above, such as about 0.50 or 0.60 mg per mg of pemetrexed diacid, or about 1.0, 1.20, 1.22, 1.4, 1.48, or 1.5 mg per mg of pemetrexed diacid. In other useful embodiments the organic base is meglumina by itself, preferably in an amount in the range 0.50 to 1.5 mg per mg of pemetrexed diacid, such as in the range of about 0.75 to about 1.10 mg per mg of pemetrexed diacid, such as about 0.80, 0.90, 0.95 or 1.00 mg per mg of pemetrexed diacid. In further useful embodiments the the organic base is diethanolamine by itself, preferably in an amount in the range from 0.50 to 0.75 mg per mg of pemetrexed diacid, such as about 0.50, 0.60 or about 0.70 mg per mg of pemetrexed diacid.

In preferred embodiments, trisodium citrate is included in the formulation. More preferably however, the citrate is added as citric acid. This has proven to further stabilise the active ingredient and prevent degradation. The citrate/citric acid is preferably added in a molar ratio to pemetrexed in the range from 1:2 to 3:1, pemetrexed to citric acid, such as more preferably in the range 1:1 to 2:1, such as about 1:1, 1:1.2 or 1:1.5. A desired ratio can be obtained with adding about 10 mg citric acid anhydrous per 25 mg of pemetrexed diacid. Preferably the citric acid or citrate is added and dissolved in solution, with the active compound base addition salt being present or prior to addition of the active ingredient and base, and then after all ingredients are in place and fully dissolved the complete solution can be suitably lyophilised to homogeneous powder if desired, which can be reconstituted, either just prior to use or to prepare a liquid product.

Preferred formulations comprise pemetrexed, trisamine and citric acid, preferably in the following relative amounts: in the range 30-40 mg trisamine per 25 mg pemetrexed diacid, preferably in the range 33-38 mg trisamine, such as about 35, about 36 or about 37 mg trisamine, and in the range of about 7.5 to 15 mg citric acid per 25 mg pemetrexed diacid, preferably in the range from 8 to 12 mg citric acid, such as about 10 mg citric acid.

Further excipients may as well be included in the formulation of the invention, such as but not limited to sugar alcohols including but not limited to mannitol, sorbitol, erythritol, or sugars such as lactose, fructose, or glucose, and pH-adjusting agents including but not limited to hydrochloric acid, sodium hydroxide, acetic acid, citric acid, malonic acid, antioxidants such as but not limited to L-cysteine, lipoic acid, dihydrolipoic acid, and methionine. It follows that these excipients shall preferably all be soluble and dissolved in the mixing formulation solutions which are generally subsequently lyophilised. Mannitol or a corresponding excipient such as an alternative sugar may be included in the preferred range of 5-50 mg/mL, such as in within the range 5-40 mg/mL, or the range 10-40 mg/mL, such as the range 10-30 mg/mL, such as about 10, 15, 20, 25, or 30 mg/mL, and preferably in an amount of 0.1-2.0 mg per mg pemetrexed, such as in the range 0.2 to 1.0 mg per mg pemetrexed and more preferably in about 1:1 weight ratio to pemetrexed acid. L-cysteine or methionine is in some embodiments added to a concentration in the range 0,05-1 mg/mL, such as in the range 0,1-0,5 mg/mL, such as 0,1 or 0,2 mg/mL or 0,5 mg/mL.

Sugar alcohols are found to be suitable in freeze dried formulations to be reconstituted. Such formulations may comprise a sugar alcohol such as but not limited to mannitol, sorbitol, erythritol, preferably in an amount corresponding to 0.5 to 2.5 mg sugar alcohol (e.g. mannitol) per mg pemetrexed diacid, such as 0.5, 1.0, or 1.5 mg per mg pemetrexed diacid.

The formulations are suitably prepared as follows: pemetrexed diacid is added to water or aqueous solution (80% of final amount of liquid); if trisodium citrate or citric acid is used in the formulation this is conveniently already dissolved in the solution prior to addition of the pemetrexed or added later, the solution is bubbled with nitrogen. The basic organic amine is added under stirring, this may be done in one or more portions, and a clear solution obtained. pH should be in the range 8,3-8,7, preferably 8,5. Water is added to reach full volume. The solution is filled in suitably sized vials (such as e.g. 5 mL vials). These may be clear polymer vials which are preferably sealed under nitrogen. It is preferred to seal using bromobutylic rubber stopper coated with fluoropolymer.

In other embodiments, the process may comprise the following: about 80% of total water is loaded in dissolution tank, the pemetrexed diacid is added, either before or after addition and dissolution of the basic organic amine (e.g. trisamine). Citric acid is added, which may or may not be predissolved, stirred until full dissolution. An antioxidant such as L-cysteine (preferably L-cysteine hydrochloride monohydrate) can be added and solution stirred further, pH is adjusted to a desired range/value, typically within a range from 7.0 to 8.0, such as 7.2 or 7.5, and the volume adjusted to full volume by adding remaining water. The solution is filtered.

A preferred formulation comprises pemetrexed diacid, trisamine, and citric acid, preferably about 25 mg/mL pemetrexed diacid, in the range of about from 35 to 38 mg/mL trisamine, and about 10 mg/mL citric acid. The formulation preferably comprises an antioxidant such as but not limited to L-cysteine, preferably in the form L-cysteine hydrochloride monohydrate, preferably in an amount of about 0,5 mg/mL. The liquid formulation should preferably have a pH in the range of about 7 to 8, such as about 7,5.

The above formulations can be lyophilised, as mentioned above. A sugar alcohol such as mannitol is preferably added to the solutions to be lyophilised.

### EXAMPLES

### Example 1. Formulations

Laboratory scale batches of sample formulations were prepared as follows:
2.5 g pemetrexed diacid was added to solution bubbled with nitrogen. In the cases where trisodium citrate and/or L-cysteine is used this was added and dissolved prior to adding the pemetrexed. The organic base was added under stirring until a solution was obtained (about 1.52 g), then remaining amount of the organic base was added, to obtain a pH of about 8,5. The solution was brought to a final volume of 100 mL. The solution was prepared under nitrogen and protected from light. The solution was filled into 5 ml clear polymer vials. The vials were sealed under nitrogen using bromobutylic rubber stopper coated with fluoropolimer.

**Liquid concentrate formulation A [12007]**

| | |
|---|---|
| Pemetrexed diacid | 25.0 mg |
| Diethanolamine | 15.0 mg |
| Trisodium citrate | 15 mg |
| Water | Up to 1 mL |

**Liquid concentrate formulation B [12008]**

| | |
|---|---|
| Pemetrexed diacid | 25.0 mg |
| Diethanolamine | 15.0 mg |
| L-Cysteine | 0.2 mg |
| Trisodium citrate | 15 mg |
| Water | Up to 1 mL |

**Liquid concentrate formulation C [13001]**

| | |
|---|---|
| Pemetrexed diacid | 25.0 mg |
| trisamine | 15.0 mg |
| Trisodium citrate | 15 mg |
| Water | Up to 1 mL |

**Liquid concentrate formulation D [13002]**

| | |
|---|---|
| Pemetrexed diacid | 25.0 mg |
| trisamine | 27.7 mg |
| Trisodium citrate | 15 mg |
| Water | Up to 1 mL |

**Liquid concentrate formulation E [13003]**

| | |
|---|---|
| Pemetrexed diacid | 25.0 mg |
| meglumine | 23.8 mg |
| Trisodium citrate | 15 mg |
| Water | Up to 1 mL |

**Liquid concentrate formulation F [13004]**

| | |
|---|---|
| Pemetrexed diacid | 25.0 mg |
| trisamine | 30.4 mg |
| Trisodium citrate | 15 mg |
| Water | Up to 1 mL |

**Liquid concentrate formulation G [12006]**

| | |
|---|---|
| Pemetrexed diacid | 25.0 mg |
| Diethanolamine | 15.0 mg |
| Water | Up to 1 mL |

**Liquid concentrate formulation H [12010]**

| | |
|---|---|
| Pemetrexed diacid | 25.0 mg |
| trisamine | 15.0 mg |
| Water | Up to 1 mL |

### Example 2. Stability test

Liquid concentrate formulations from Example 1 were stored at different temperatures for varying time periods and impurities were assayed with HPLC assay, the values are estimated concentrations as percentage based on initial content of active ingredient (100%).

**Impurities forming during storage for 2.5 weeks, 1, 2 and 3 months - formulation A**

| time/ impurity* | 2-8°C | | | | 25°C | | | | 40°C | | | |
|---|---|---|---|---|---|---|---|---|---|---|---|---|
| | 2,5w | 1m | 2m | 3m | 2,5w | 1m | 2m | 3m | 2,5w | 1m | 2m | 3m |
| u1 | 0,01 | 0,02 | 0,04 | 0,06 | 0,05 | 0,07 | 0,18 | 0,30 | 0,20 | 0,48 | 1,55 | - |
| u2 | 0,02 | 0,03 | 0,06 | 0,09 | 0,07 | 0,13 | 0,31 | 0,49 | 0,47 | 0,78 | 1,07 | - |
| u3 | 0,04 | 0,05 | 0,09 | 0,12 | 0,13 | 0,22 | 0,59 | 1,01 | 1,21 | 2,51 | 5,83 | - |
| u4 | 0,01 | 0,09 | 0,16 | - | 0,08 | 0,31 | 0,71 | 1,16 | 1,34 | 2,67 | 6,05 | - |

| | | | | | | | | | | | | |
|---|---|---|---|---|---|---|---|---|---|---|---|---|
| *unidentified impurity. | | | | | | | | | | | | |

**Impurities forming during storage for 2.5 weeks, 1, 2 and 3 months - formulation B**

| time/ impurity* | 2-8°C | | | | 25°C | | | | 40°C | | | |
|---|---|---|---|---|---|---|---|---|---|---|---|---|
| | 2,5w | 1m | 2m | 3m | 2,5w | 1m | 2m | 3m | 2,5w | 1m | 2m | 3m |
| u1 | 0,01 | 0,04 | 0,15 | 0,24 | 0,05 | 0,10 | 0,21 | 0,35 | 0,19 | 0,27 | 0,83 | |
| u2 | 0,02 | 0,10 | 0,31 | 0,49 | 0,11 | 0,23 | 0,42 | 0,62 | 0,39 | 0,43 | 0,68 | |
| u3 | 0,03 | 0,07 | 0,19 | 0,28 | 0,08 | 0,28 | 0,61 | 1,06 | 1,11 | 1,73 | 4,59 | |
| u4 | 0,01 | 0,11 | 0,26 | | 0,02 | 0,36 | 0,74 | 1,20 | 1,23 | 1,88 | 4,82 | |

| | | | | | | | | | | | | |
|---|---|---|---|---|---|---|---|---|---|---|---|---|
| *unidentified impurity. | | | | | | | | | | | | |

**Impurities forming during storage for 3 weeks, 1 month, 2 months - formulation C**

| time/ impurity* | 2-8°C | | | | 25°C | | | | 40°C | | | |
|---|---|---|---|---|---|---|---|---|---|---|---|---|
| | 3w | 1m | 2m | 3m | 3w | 1m | 2m | 3m | 3w | 1m | 2m | 3m |
| u1 | 0,05 | 0,12 | 0,22 | - | 0,26 | 0,39 | 1,30 | - | 1,55 | 1,71 | 3,80 | - |
| u2 | 0,10 | 0,22 | 0,40 | - | 0,53 | 0,80 | 2,57 | - | 2,96 | 3,15 | 4,11 | - |
| u3 | 0,01 | 0,08 | 0,12 | - | 0,22 | 0,31 | 0,88 | - | 2,45 | 2,66 | 11,5 | - |
| u4 | 0,05 | 0,08 | - | - | 0,23 | 0,32 | 0,90 | - | 2,49 | 2,71 | 11,7 | - |

| | | | | | | | | | | | | |
|---|---|---|---|---|---|---|---|---|---|---|---|---|
| *unidentified impurity. | | | | | | | | | | | | |

**Impurities forming during storage for 2 weeks and 1 month - formulation D**

| time/ impurity* | 2-8°C | | | | 25°C | | | | 40°C | | | |
|---|---|---|---|---|---|---|---|---|---|---|---|---|
| | 2w | 1m | | | 2w | 1m | | | 2w | 1m | | |
| u1 | 0,01 | 0,01 | - | - | 0,02 | 0,05 | - | - | 0,07 | 0,43 | - | - |
| u2 | 0,02 | 0,03 | - | - | 0,05 | 0,15 | - | - | 0,30 | 1,06 | - | - |
| u3 | 0,01 | 0,03 | - | - | 0,05 | 0,19 | - | - | 0,57 | 3,42 | - | - |
| u4 | - | - | - | - | 0,06 | 0,19 | - | - | 0,59 | 3,49 | - | - |

| | | | | | | | | | | | | |
|---|---|---|---|---|---|---|---|---|---|---|---|---|
| *unidentified impurity. | | | | | | | | | | | | |

**Impurities forming during storage for 2 weeks and 1 month - formulation E**

| time/ impurity* | 2-8°C | | | | 25°C | | | | 40°C | | | |
|---|---|---|---|---|---|---|---|---|---|---|---|---|
| | 2w | 1m | 2m | 3m | 2w | 1m | 2m | 3m | 2w | 1m | 2m | 3m |
| u1 | 0,004 | 0,01 | - | - | 0,01 | 0,06 | - | - | 0,08 | 0,81 | - | - |
| u2 | 0,01 | 0,02 | - | - | 0,04 | 0,15 | - | - | 0,28 | 1,17 | - | - |
| u3 | 0,01 | 0,02 | - | - | 0,06 | 0,19 | - | - | 0,66 | 5,12 | - | - |
| u4 | - | - | - | - | 0,06 | 0,20 | - | - | 0,67 | 5,19 | - | - |

| | | | | | | | | | | | | |
|---|---|---|---|---|---|---|---|---|---|---|---|---|
| *unidentified impurity. | | | | | | | | | | | | |

**Impurities forming during storage for 2 weeks and 1 month - formulation F**

| time/ impurity* | 2-8°C | | | | 25°C | | | | 40°C | | | |
|---|---|---|---|---|---|---|---|---|---|---|---|---|
| | t0 | 2w | 1m | 2m | 2w | 1m | 2m | 3m | 2w | 1m | 2m | 3m |
| u1 | - | 0,01 | 0,01 | - | 0,02 | 0,04 | - | - | 0,07 | 0,17 | - | - |
| u2 | 0,01 | 0,02 | 0,03 | - | 0,06 | 0,12 | - | - | 0,26 | 0,49 | - | - |
| u3 | - | 0,03 | 0,04 | - | 0,08 | 0,17 | - | - | 0,56 | 1,40 | - | - |
| u4 | 0,05 | 0,06 | 0,06 | - | 0,11 | 0,20 | - | - | 0,60 | 1,45 | - | - |

| | | | | | | | | | | | | |
|---|---|---|---|---|---|---|---|---|---|---|---|---|
| *unidentified impurity. | | | | | | | | | | | | |

**Impurities forming during storage for 2.5 weeks, 1, 2 and 3 months - formulation G**

| time/ impurity* | 2-8°C | | | | 25°C | | | | 40°C | | | |
|---|---|---|---|---|---|---|---|---|---|---|---|---|
| | 2,5w | 1m | 2m | 3m | 2,5w | 1m | 2m | 3m | 2,5w | 1m | 2m | 3m |
| u1 | 0,41 | 0,92 | 2,18 | 3,06 | 0,52 | 0,99 | 2,35 | 2,06 | 0,10 | 1,93 | 2,65 | - |
| u2 | 0,86 | 1,66 | 3,91 | 5,32 | 1,20 | 1,94 | 2,55 | 1,62 | 1,29 | 0,80 | 0,18 | - |
| u3 | 0,33 | 0,65 | 1,75 | 2,48 | 0,82 | 1,49 | 3,31 | 2,94 | 4,39 | 8,66 | 14,9 | - |
| u4 | 0,36 | 0,71 | 1,88 | 2,63 | 0,89 | 1,60 | 3,48 | 3,08 | 4,57 | 8,91 | 15,2 | - |

| | | | | | | | | | | | | |
|---|---|---|---|---|---|---|---|---|---|---|---|---|
| *unidentified impurity. | | | | | | | | | | | | |

**Impurities forming during storage for 2.5 weeks, 1, 2 and 3 months - formulation H**

| time/ impurity* | 2-8°C | | | | 25°C | | | | 40°C | | | |
|---|---|---|---|---|---|---|---|---|---|---|---|---|
| | 2,5w | 1m | 2m | 3m | 2,5w | 1m | 2m | 3m | 2,5w | 1m | 2m | 3m |
| u1 | 0,28 | 0,62 | 1,40 | 1,74 | 0,61 | 1,05 | 2,85 | 3,85 | 0,94 | 2,04 | 2,49 | - |
| u2 | 0,49 | 1,10 | 2,40 | 2,94 | 0,43 | 1,81 | 3,23 | 3,33 | 1,58 | 1,89 | 0,98 | - |
| u3 | 0,12 | 0,26 | 0,56 | 0,70 | 0,34 | 0,62 | 2,24 | 3,53 | 1,38 | 4,39 | 11,9 | - |
| u4 | - | 0,26 | 0,57 | 0,71 | 0,35 | 0,64 | 2,28 | 3,59 | 1,40 | 4,44 | 12,1 | - |

| | | | | | | | | | | | | |
|---|---|---|---|---|---|---|---|---|---|---|---|---|
| *unidentified impurity. | | | | | | | | | | | | |

### Example 3. Further formulations

Further formulations have been developed and tested.

**Liquid concentrate formulation I [13008]**

| | |
|---|---|
| Pemetrexed diacid | 25.0 mg/mL |
| trisamine | 35.2 mg/mL |
| Citric acid anhydrous | 10 mg/mL |
| Water | Up to 1 mL |

**Liquid concentrate formulation J [13009]**

| | |
|---|---|
| Pemetrexed diacid | 25.0 mg/mL |
| trisamine | 37 mg/mL |
| Citric acid anhydrous | 10 mg/mL |
| L-Cysteine hydrochloride monohydrate | 0.5 mg/mL |
| Water | Up to 1 mL |

The formulations were prepared as follows:
Nitrogen was bubbled through water prior to use, for 2 hours. Approx. 80% of the total batch water amount was loaded in stainless steel dissolution tank. Pemetrexed diacid was added and stirred. Citric acid anhydrous powder was added, followed by trisamine (corresponding to 35 mg/mL), solution bubbled with nitrogen, stirred for approx. 5 minutes, until dissolution. (pH 7.14.) L-Cysteine HCl monohydrate added, followed by small quantities of trisamine until pH reached 7.50. Finally water was added to full volume. Solution filtered twice before transfer into vials.

**Liquid concentrate formulation K [SP01]**

| | |
|---|---|
| Pemetrexed diacid | 25.0 mg/mL |
| trisamine | 35 mg/mL |
| Citric acid anhydrous | 10 mg/mL |
| L-Cysteine hydrochloride monohydrate | 0.5 mg/mL |
| Trisamine (as aq soln. 5 mol/L) | To pH 7.5 |
| Water | Up to 1 mL |

The formulations are prepared as follows:
Approx. 80% of the total batch water amount was loaded in stainless steel dissolution tank and stirred under nitrogen. Trisamine is loaded through glass predissolutor, stirred for about 5 min. under nitrogen until completely dissolved. Pemetrexed diacid is added to through predissolution tank and stirred for not less than 5 min. A solution of citric acid in water is added through the predissolution tank, stirred for about 5 min. until complete dissolution. Solution of L-cysteine hydrochloride monohydrate in water is added to predissolution tank and stirred for about 5 min. The pH of the solution is adjusted to 7.5 with a solution of 5N trisamine. Water is added to final weight.

The solution is filtered with 0.45 µm porous filter, and sterilized with sterilisation filtration through 0.22 µm filter. The filtered solution is collected is sterilised tank.

### Example 4. Further stability tests

Liquid concentrate formulations from Example 3 are stored at different temperatures for varying time periods and impurities are assayed with HPLC assay, the values are estimated concentrations as percentage based on initial content of active ingredient (100%). The samples are stored at 2-8°C, 25°C (60% RH), 30°C (75% RH) and 40°C (75% RH), respectively, for time periods of 2,5 weeks, 1 month, 2 months, and 3 months.

Long term stability studies are conducted for 24 and 36 months.

### Example 5. Lyophilised formulations

**Formulation L [2.1.1604-13001]**

| | |
|---|---|
| Pemetrexed diacid | 100 mg |
| trisamine | 120 mg |
| mannitol | 100 mg |
| triasamine | To pH 8.5 |
| water | Up to 4.2 mL* |

| | |
|---|---|
| *overfilling of 5%. | |

**Formulation M [2.1.1604-13002]**

| | |
|---|---|
| Pemetrexed diacid | 100 mg |
| trisamine | 60 mg |
| mannitol | 100 mg |
| triasamine | To pH 7.2 |
| water | Up to 4.2 mL* |

**Formulation N [2.2.0205-13004]**

| | |
|---|---|
| Pemetrexed diacid | 100 mg |
| trisamine | 60 mg |
| mannitol | 100 mg |
| triasamine | To pH 7.2 |
| water | Up to 4.00 mL |

**Formulation O [2.2.0205-13005]**

| | |
|---|---|
| Pemetrexed diacid | 100 mg |
| trisamine | 60 mg |
| mannitol | 50 mg |
| triasamine | To pH 7.2 |
| water | Up to 4.00 mL* |

**Formulation P [2.2.0205-13006]**

| | |
|---|---|
| Pemetrexed diacid | 100 mg |
| trisamine | 60 mg |
| mannitol | 0 mg |
| triasamine | To pH 7.2 |
| water | Up to 4.00 mL* |

**Formulation Q [2.3.2008-13008]**

| | |
|---|---|
| Pemetrexed diacid | 100 mg |
| trisamine | 60 mg |
| mannitol | 0 mg |
| | pH 6.60 |
| water | Up to 4.00 mL* |

**Formulation Q [2.3.2008-13008]**

| | |
|---|---|
| Pemetrexed diacid | 100 mg |
| trisamine | 60 mg |
| mannitol | 0 mg |
| | pH 6.60 |
| water | Up to 4.00 mL* |

The formulations were prepared as follows: approx. 80% of the total amount of water loaded in preparation vessel, at a temperature of maximum 25°C. Filtered sterile nitrogen bubbled water for 20 minutes.

Weighted quantity of trisamine quantitatively transferred into the preparation vessel using successive rinses with water for injection.

Stirred until complete dissolution, about 5 minutes. Filtered sterile nitrogen was continued bubbled in solution.

Weighted quantity of Pemetrexed diacid transferred into the preparation vessel using successive rinses with water for injection. Stirred until complete dissolution, about 10 minutes. Filtered sterile nitrogen was continued bubbled in solution.

Quantitatively transferred into the preparation vessel the weighted Mannitol quantity using water for injection. Stirred for about 10 minutes, until complete dissolution. Filtered sterile nitrogen was continued bubbled in solution.

pH was adjusted to desired value 7.2 (7.0 - 7.4) with hydrochloric acid 0.1 mol/L solution or trisamine 0.1 mol/L solution. Complete to final weight with water for injection and continue stirring for about 5 minutes to homogenize the final solution with continued bubbled nitrogen into solution.

The solution was filtered through sterilizing filter of 0.2 µm porosity. The filtered solution is divided into the vials. After filling the vials are partially stoppered with rubber closure for freeze-dried, transferred into the freeze-drier and freeze-dried.

### Example 6. Stability tests

The lyophilised formulations are stored in sealed vials at 25°C, 40° and 56°C, at 60% RH (surrounding vials). Stored for 2 weeks, 2.5 weeks, 3 weeks, or 3 or 6 months at 25°C or 40°; also 56°C for 2 and 3 weeks. After storage the powder samples are reconstituted (to 25 mg/mL) and samples analysed for impurites as described above (Example 2).

## Claims

1. A pharmaceutical formulation comprising pemetrexed diacid and one or more basic organic amine selected from the group consisting of diethanolamine, tris(hydroxymethyl)aminomethane, and meglumine.

2. The pharmaceutical formulation of claim 1, wherein said basic organic amine is tris(hydroxymethyl)aminomethane.

3. The pharmaceutical formulation of claim 1 or 2, comprising said one or more basic organic amine in a total amount in the range corresponding to 1.0 to 2.0 mg per mg of pemetrexed diacid, and preferably in the range corresponding to 1.0 to 1.5 mg per mg of pemetrexed diacid.

4. The pharmaceutical formulation of any of claims 1 to 3, which is a liquid concentrate formulation, to be diluted prior to use in a suitable infusion solution.

5. The pharmaceutical formulation of claim 4, comprising pemetrexed in a concentration in the range of about 2 to 80 mg/mL, and more preferably in the range from about 5 to about 50 mg/mL, and most preferably about 25 m/mL.

6. The pharmaceutical formulation of any of claims 1 to 3, which is a dry powder formulation, to be reconstituted in solution prior to use.

7. The pharmaceutical formulation of any of the preceding claims, further comprising an excipient selected from citric acid, trisodium citrate, mannitol, sorbitol, lactose, fructose, glucose, methionine, and L-cysteine.

8. The pharmaceutical formulation of any of the preceding claims, prepared by dissolving all ingredients to obtain a liquid formulation, which is optionally lyophilised to dry powder.

9. The pharmaceutical formulation of any one of claims 1 to 5 and 7 to 8, comprising in the range of about 10-50 mg/mL pemetrexed diacid, in the range 15-70 mg/mL trisamine, in the range 5-20 mg/mL citric acid anhydrous, or in the range 0,25 - 1,0 mg/mL L-cysteine, the liquid formulation having a pH in the range 7.0-8.0.

10. The pharmaceutical formulation of any one of claims 1 to 5 and 7 to 8, comprising about 25 mg/mL pemetrexed diacid, in the range 35-38 mg/mL trisamine, about 10 mg/mL citric acid anhydrous, and about 10 mg/mL L-cysteine, the liquid formulation having a pH in the range 7.2-7.5, preferably pH 7.5.

11. A process for preparing a pharmaceutical formulation comprising pemetrexed, comprising the steps of
- dissolving in water or aqueous solution pemetrexed diacid,
- dissolving an organic base selected from the group consisting of diethanolamine, tris(hydroxymethyl)aminomethane, and meglumine in the solution,
- adding water to obtain final desired volume.

12. The process of claim 11, further comprising dissolving in the solution citric acid.

13. The process of claim 11, wherein said organic base is tris(hydroxymethyl)-aminomethane.

14. The process of any of claims 11 to 13, further comprising adjusting the pH to a value in the range 7-8 and preferably pH 7.5.

15. The process of any of claims 11 to 14, further comprising a step of lyophilising the obtained solution, in order to obtain a dry powder formulation, to be reconstituted in water or aqueous solution prior to use.
